# EUROPEAN PATENT SPECIFICATION

(11) **EP 4 643 768 B1**
(45) Date of publication and mention of the grant of the patent: **02.12.2026**
(21) Application number: 23912566.9
(22) Date of filing: 14.11.2023
(51) Int. Cl.: G06T 7/00, A61B 5/00, A61B 5/02, A61B 5/055, G16H 30/20, G16H 30/40, G16H 50/20

(54) **DEVICE, METHOD, SYSTEM AND PROGRAM FOR ESTIMATING TIME OF OCCURRENCE OF ACUTE CEREBRAL INFARCTION**
VORRICHTUNG, VERFAHREN, SYSTEM UND PROGRAMM ZUR SCHÄTZUNG DER ZEIT DES AUFTRETENS EINES AKUTEN HIRNINFARKTS
DISPOSITIF, PROCÉDÉ, SYSTÈME ET PROGRAMME POUR ESTIMER LE TEMPS DE SURVENUE D'UN INFARCTUS CÉRÉBRAL AIGU

(30) Priority: 26.12.2022 KR 20220184041; 31.01.2023 KR 20230012380
(43) Date of publication of application: 05.11.2025
(73) Proprietor: NUNAPS INC., Seoul 05505 (KR)
(72) Inventor: KANG, Dong Wha, Seoul 05505 (KR); LEE, Eun Jae, Seoul 06521 (KR); KANG, Dong Won, Seoul 08703 (KR); KIM, Young Sun, Seongnam-si, Gyeonggi-do 13445 (KR)
(74) Representative: dompatent
(86) International application number: PCT/KR2023/018219
(87) International publication number: WO 2024/143889

(56) References cited:
- WO-A1-2018/093189
- KR-A- 20190 068 254
- POLSON JENNIFER ET AL: "A Semi-Supervised Learning Framework to Leverage Proxy Information for Stroke MRI Analysis", 2021 43RD ANNUAL INTERNATIONAL CONFERENCE OF THE IEEE ENGINEERING IN MEDICINE & BIOLOGY SOCIETY (EMBC), IEEE, 1 November 2021 (2021-11-01), pages 2258 - 2261, XP034043297, DOI: 10.1109/EMBC46164.2021.9631098
- LEE HYUNNA ET AL: "Machine Learning Approach to Identify Stroke Within 4.5 Hours", STROKE, vol. 51, no. 3, 1 March 2020 (2020-03-01), US, pages 860 - 866, XP093188516, ISSN: 0039-2499, DOI: 10.1161/STROKEAHA.119.027611
- MINA PETKOVA ET AL: "MR imaging helps predict time from symptom onset in patients with acute stroke: implications for patients with unknown onset time", RADIOLOGY, 1 December 2010 (2010-12-01), United States, pages 782 - 792, XP055521852, Retrieved from the Internet <URL:https://pubs.rsna.org/doi/pdf/10.1148/radiol.10100461> DOI: 10.1148/radiol.10100461
- HAOYUE ZHANG ET AL: "Intra-Domain Task-Adaptive Transfer Learning to Determine Acute Ischemic Stroke Onset Time", ARXIV.ORG, CORNELL UNIVERSITY LIBRARY, 201 OLIN LIBRARY CORNELL UNIVERSITY ITHACA, NY 14853, 5 November 2020 (2020-11-05), XP081915099, DOI: 10.1016/J.COMPMEDIMAG.2021.101926
- LEE HYUNNA, LEE EUN-JAE, HAM SUNGWON, LEE HAN-BIN, LEE JI SUNG, KWON SUN U., KIM JONG S., KIM NAMKUG, KANG DONG-WHA: "Machine Learning Approach to Identify Stroke Within 4.5 Hours", STROKE, LIPPINCOTT WILLIAMS & WILKINS, US, vol. 51, no. 3, 1 March 2020 (2020-03-01), US , pages 860 - 866, XP093188516, ISSN: 0039-2499, DOI: 10.1161/STROKEAHA.119.027611
- ZHU HAICHEN, JIANG LIANG, ZHANG HONG, LUO LIMIN, CHEN YANG, CHEN YUCHEN: "An automatic machine learning approach for ischemic stroke onset time identification based on DWI and FLAIR imaging", NEUROIMAGE: CLINICAL, vol. 31, 1 January 2021 (2021-01-01), pages 102744, XP093188518, ISSN: 2213-1582, DOI: 10.1016/j.nicl.2021.102744
- GROSSER MALTE, GELLISSEN SUSANNE, BORCHERT PATRICK, SEDLACIK JAN, NAWABI JAWED, FIEHLER JENS, FORKERT NILS D.: "Localized prediction of tissue outcome in acute ischemic stroke patients using diffusion- and perfusion-weighted MRI datasets", PLOS ONE, PUBLIC LIBRARY OF SCIENCE, US, vol. 15, no. 11, 5 November 2020 (2020-11-05), US , pages e0241917, XP093188527, ISSN: 1932-6203, DOI: 10.1371/journal.pone.0241917

## Description

### [TECHNICAL FIELD]

The present disclosure relates to a method, device, system, and program for predicting an occurrence time of acute cerebral infarction.

### [BACKGROUND ART]

Thrombolysis, which helps to open cerebral blood vessels blocked by thrombi or embolism and prevents damage to brain cells, is widely known as a method for treating patients with acute cerebral infarction. Since thrombolysis needs to be performed quickly before brain cells are damaged, it needs to be performed within a short period of time from the onset of cerebral infarction.

However, most patients come to the hospital without knowing the occurrence time of acute cerebral infarction. In other words, they are discovered by others after collapsing and transferred to the hospital, so it is impossible to confirm how long before they are discovered by others that they have acute cerebral infarction. In such cases, there is a lack of specialists who can determine whether a patient can undergo thrombolysis based solely on medical imaging. In addition, inexperienced medical staff often work in emergency rooms where patients with acute cerebral infarction visit at an emergency.

Therefore, it is necessary to provide information so that medical staff can safely and quickly determine the occurrence time of acute cerebral infarction. A similar device, method, and system is known from POLSON JENNIFER ET AL: "A Semi-Supervised Learning Framework to Leverage Proxy Information for Stroke MRI Analysis", disclosing a semi-supervised learning framework for stroke MRI analysis. Specifically, POLSON JENNIFER ET AL discloses training a deep learning model on MRI to classify tissue clock and leveraging time clock as a weak proxy label to supplement training in a semi-supervised learning framework to detect DWI-FLAIR mismatch in an automated fashion. Furthermore, it discloses receiving patient MR imaging, classifying the image into a fluid-attenuated inversion recovery (FLAIR) image and a diffusion-weighted image (DWI), aligning the images via registration to an anatomical atlas, detecting an infarction region, and outputting a probability prediction value for the occurrence time.

### [DETAILED DESCRIPTION OF THE INVENTION]

### [TECHINICAL PROBLEM]

The embodiments disclosed in the present disclosure can provide information that allows medical staff to safely and quickly determine the occurrence time of acute cerebral infarction.

Technical problems of the inventive concept are not limited to the technical problems mentioned above, and other technical problems not mentioned will be clearly understood by those skilled in the art from the following description.

### [TECHNICAL SOLUTION]

The invention is defined by the claims. In an aspect of the present disclosure, a device for predicting an occurrence time of acute cerebral infarction may include a communication module configured to perform communication with an imaging device that captures an image of an acute cerebral infarction patient; and a processor configured to control an operation related to prediction of the occurrence time of acute cerebral infarction, wherein the processor is configured to: receive the image of the acute cerebral infarction patient from the imaging device through the communication module, classify the image into a first image corresponding to a fluid-attenuated inversion recovery image and a second image corresponding to a diffusion-weighted image, acquire the first image and the second image, align the first image and the second image with respect to an MNI region corresponding to an activated region of a brain, detect an infarction region in the second image, and output a probability prediction value for the occurrence time of acute cerebral infarction based on the first image, the second image, and the infarction region.

Furthermore, the processor may be configured to perform learning based on machine learning based on the first image, the second image, and the infarction region, and output the probability prediction value for the occurrence time of acute cerebral infarction analyzed by learning based on the machine learning.

Furthermore, the processor may be configured to standardize an intensity of the second image, perform learning based on machine learning based on the standardized second image, and detect the infarction region in the second image analyzed by learning based on the machine learning.

Furthermore, the processor may be configured to label an area of the infarction region, perform learning based on machine learning based on the first image, the second image, and the area of the infarction region, and output the probability prediction value for the time of acute cerebral infarction occurrence analyzed by learning based on the machine learning.

Furthermore, the processor may be configured to further generate reference data based on the machine learning based on the first image and the second image.

In another aspect of the present disclosure, a method for predicting an occurrence time of acute cerebral infarction performed by a prediction device may include receiving, by a communication module of the prediction device, an image of an acute cerebral infarction patient from an imaging device; classifying, by a processor of the prediction device, the image into a first image corresponding to a fluid-attenuated inversion recovery image and a second image corresponding to a diffusion-weighted image; acquiring, by the processor, the first image and the second image; aligning, by the processor, the first image and the second image with respect to an MNI region corresponding to an activated region of a brain; detecting, by the processor, an infarction region in the second image; and outputting, by the processor, a probability prediction value for the occurrence time of acute cerebral infarction based on the first image, the second image, and the infarction region.

In still another aspect of the present disclosure, a system for predicting an occurrence time of acute cerebral infarction may include an imaging device configured to acquiring an image of an acute cerebral infarction patient; and a prediction device for predicting the occurrence time of acute cerebral infarction configured to perform communication with the imaging device, wherein the prediction device is configured to: receive the image of the acute cerebral infarction patient from the imaging device, classify the image into a first image corresponding to a fluid-attenuated inversion recovery image and a second image corresponding to a diffusion-weighted image, acquire the first image and the second image, align the first image and the second image with respect to an MNI region corresponding to an activated region of a brain, detect an infarction region in the second image, and output a probability prediction value for the occurrence time of acute cerebral infarction based on the first image, the second image, and the infarction region.

In addition, a computer program stored in a computer-readable recording medium may be further provided to perform a method for predicting the golden hour at the occurrence time of acute cerebral infarction by being combined with a computer as hardware.

In addition, a computer-readable recording medium recording a computer program for executing a method for implementing the present disclosure may be further provided.

### [ ADVANTAGEOUS EFFECTS OF THE INVENTION]

According to the present disclosure, it is provided the effect of being able to safely and quickly provide information that allows medical staff to determine the occurrence time of acute cerebral infarction.

The effects of the present disclosure are not limited to the effects mentioned above, and other effects not mentioned can be clearly understood by those skilled in the art from the description.

### [ DESCRIPTION OF THE DRAWINGS]

FIG. 1 is a diagram illustrating an example of a system for predicting an occurrence time of acute cerebral infarction according to the present disclosure.
FIG. 2 illustrates a configuration of a prediction device of FIG. 1.
FIGS. 3 and 4 are diagrams illustrating an example of a process of outputting a probability prediction value for an occurrence time of acute cerebral infarction by the processor of FIG. 2.
FIG. 5 is a diagram illustrating another example of a system for predicting an occurrence time of acute cerebral infarction according to the present disclosure.
FIG. 6 is a diagram illustrating an example of a process for outputting a probability prediction value for an occurrence time of acute cerebral infarction in the output step of FIG. 5.

### [ BEST MODE]

In the drawings, the same reference numeral refers to the same element. This disclosure does not describe all elements of embodiments, and general contents in the technical field to which the present disclosure belongs or repeated contents of the embodiments will be omitted. The terms, such as "unit, module, member, and block" may be embodied as hardware or software, and a plurality of "units, modules, members, and blocks" may be implemented as one element, or a unit, a module, a member, or a block may include a plurality of elements.

Throughout this specification, when a part is referred to as being "connected" to another part, this includes "direct connection" and "indirect connection", and the indirect connection may include connection via a wireless communication network. Furthermore, when a certain part "includes" a certain element, other elements are not excluded unless explicitly described otherwise, and other elements may in fact be included.

Furthermore, when a certain part "includes" a certain element, other elements are not excluded unless explicitly described otherwise, and other elements may in fact be included.

In the entire specification of the present disclosure, when any member is located "on" another member, this includes a case in which still another member is present between both members as well as a case in which one member is in contact with another member.

The terms "first," "second," and the like are just to distinguish an element from any other element, and elements are not limited by the terms.

The singular form of the elements may be understood into the plural form unless otherwise specifically stated in the context.

Identification codes in each operation are used not for describing the order of the operations but for convenience of description, and the operations may be implemented differently from the order described unless there is a specific order explicitly described in the context.

Hereinafter, operation principles and embodiments of the present disclosure will be described with reference to the accompanying drawings.

In this specification, the prediction device according to the present disclosure includes various devices that may perform computational processing and provide results to a user. For example, the device according to the present disclosure may include a computer, a server device, and a portable terminal, or may be in the form of one of them.

Here, the computer may include, for example, a notebook, desktop, laptop, tablet PC, slate PC, and the like. equipped with a web browser.

The server device is a server that communicates with an external device to process information, and may include an application server, a computing server, a database server, a file server, a game server, a mail server, a proxy server, and a web server.

The portable terminal may include, for example, a wireless communication device that ensures portability and mobility, such as a PCS (Personal Communication System), a GSM (Global System for Mobile communications), a PDC (Personal Digital Cellular), a PHS (Personal Handyphone System), a PDA (Personal Digital Assistant), an IMT (International Mobile Telecommunication)-2000, a CDMA (Code Division Multiple Access)-2000, a WCDMA (W-Code Division Multiple Access), a WiBro (Wireless Broadband Internet) terminal, a smart phone, and all kinds of handheld-based wireless communication devices, and wearable devices such as a watch, a ring, a bracelet, an anklet, a necklace, glasses, contact lenses, or a head-mounted device (HMD).

A system for predicting an occurrence time of acute cerebral infarction according to the present disclosure may be provided to receive an image of an acute cerebral infarction patient from an imaging device, classify the image into a first image corresponding to a fluid-attenuated inversion recovery image and a second image corresponding to a diffusion-weighted image, acquire the first image and the second image, align the first image and the second image with respect to an MNI region corresponding to an activated region of a brain, detect an infarction region in the second image, and output a probability prediction value for the occurrence time of acute cerebral infarction based on the first image, the second image, and the infarction region.

The system for predicting an occurrence time of acute cerebral infarction according to the present disclosure may provide information so that medical staff may safely and quickly determine the occurrence time of acute cerebral infarction.

Hereinafter, the system for predicting an occurrence time of acute cerebral infarction according to the present disclosure will be described in detail.

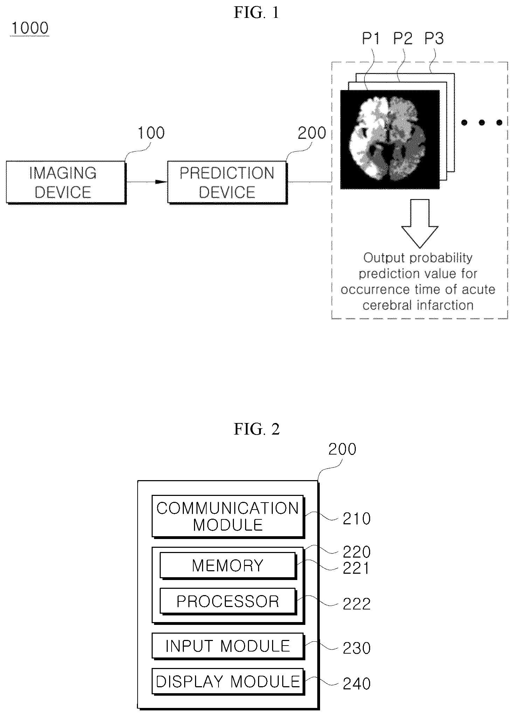

FIG. 1 is a diagram illustrating an example of a system for predicting an occurrence time of acute cerebral infarction according to the present disclosure. FIG. 2 illustrates a configuration of a prediction device of FIG. 1.

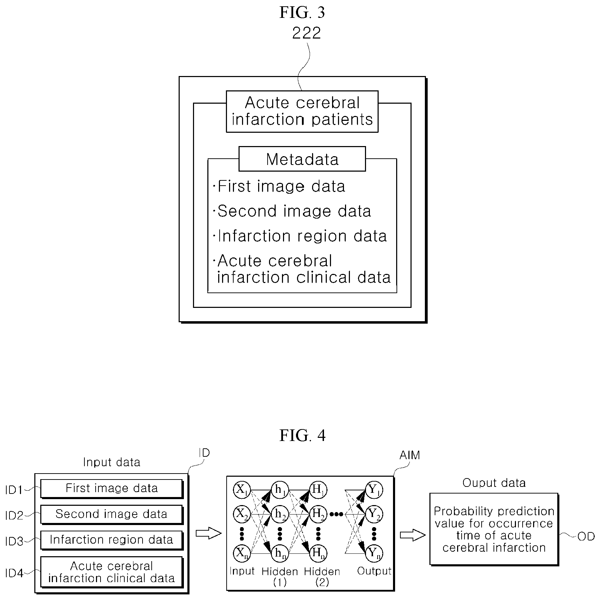

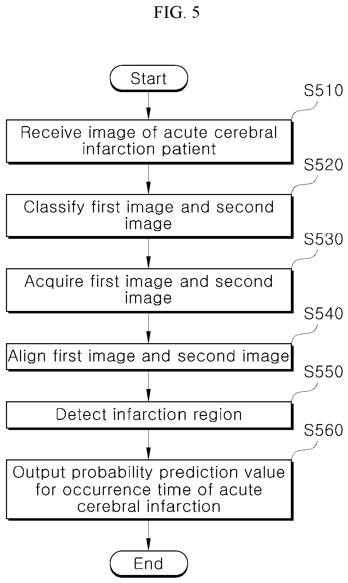

FIGS. 3 and 4 are diagrams illustrating an example of a process of outputting a probability prediction value for an occurrence time of acute cerebral infarction by the processor of FIG. 2. FIG. 5 is a diagram illustrating another example of a system for predicting an occurrence time of acute cerebral infarction according to the present disclosure.

Referring to FIGS. 1 to 5, a system 1000 may include an imaging device 100 and a prediction device 200.

The imaging device 100 may acquire an image of an acute cerebral infarction patient and transmit it to the prediction device 200. At this time, the imaging device 100 may acquire a magnetic resonance imaging (MRI) of the acute cerebral infarction patient and transmit it to the prediction device 200. The prediction device 200 may predict an occurrence time of acute cerebral infarction for the acute cerebral infarction patient. Here, the prediction device 200 may output a probability prediction value for the occurrence time of acute cerebral infarction. In this case, the prediction device 200 may include a communication module 210 and a controller 220.

The communication module 210 may communicate with the imaging device 100 that captures the image of the acute cerebral infarction patient. The communication module 210 may receive the images of the acute cerebral infarction patient obtained from the imaging device 100. The communication module 210 may include at least one of a wired communication module and a wireless communication module.

The wired communication module may include various wired communication modules such as a Local Area Network (LAN) module, a Wide Area Network (WAN) module, or a Value Added Network (VAN) module, as well as various cable communication modules such as a Universal Serial Bus (USB), a High Definition Multimedia Interface (HDMI), a Digital Visual Interface (DVI), RS-232 (recommended standard232), power line communication, or a plain old telephone service (POTS).

The wireless communication module may include a wireless communication module that supports various wireless communication methods such as a WiFi module, a WiBro (Wireless broadband) module, GSM (Global System for Mobile Communication), CDMA (Code Division Multiple Access), WCDMA (Wideband Code Division Multiple Access), UMTS (Universal Mobile Telecommunications System), TDMA (Time Division Multiple Access), LTE (Long Term Evolution), 4G, 5G, and 6G.

The controller 220 may include a memory 221 and a processor 222.

The memory 221 may store data on an algorithm for controlling the operation of components within the device or a program that reproduces the algorithm. The processor 222 may perform the aforementioned operation using the data stored in the memory 221. Here, the memory 221 and the processor 222 may each be implemented as separate chips. In addition, the memory 221 and the processor 222 may be implemented as a single chip.

The memory 221 may store data supporting various functions of the device, programs for the operation of components within the device, data input/output, and a plurality of application programs application programs or applications run on the device, data for the operation of the device, and commands. At least some of these application programs may be downloaded from an external server via wireless communication.

The memory 221 may include at least one type of storage medium among a flash memory type, a hard disk type, an SSD type (Solid State Disk type), an SDD type (Silicon Disk Drive type), a multimedia card micro type, a card type memory (e.g., an SD or XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk.

The memory 221 may store images P1, P2, P3, ... of the acute cerebral infarction patient. The memory 221 may store data related to a prediction of the occurrence time of acute cerebral infarction.

The processor 222 may control operations related to the prediction of the occurrence time of acute cerebral infarction. The processor 222 may receive images P1, P2, P3, ... of the acute cerebral infarction patient from the imaging device 100 by the communication module 210, classify the images P1, P2, P3, ... into a first image corresponding to a fluid-attenuated inversion recovery (FLAIR) image and a second image corresponding to a diffusion weighted image (DWI), and acquire the first image and the second image. In this case, the first image and the second image may be acute cerebral infarction patients whose acute cerebral infarction occurrence time is not confirmed.

The processor 222 may align the first image and the second image with the MNI (Montreal Neulological Institute) region corresponding to the activated region of the brain, and may detect the infarction region in the second image. Here, the MNI region may include the MNI coordinate value output as the result value for the activated region of the brain when analyzing the brain image. At this time, the infarction region may be an area where the blood vessel is narrowed or blocked by a blood clot or embolism, causing a disruption in normal blood supply to the growing tissue.

The memory 221 may store a probability prediction value for the occurrence time of acute cerebral infarction that is learned and output based on machine learning. Referring to FIG. 4, the processor 222 may learn input values of first image data ID1 corresponding to fluid-attenuated inversion recovery image, which is metadata, second image data ID2 corresponding to the diffusion-weighted image, infarct area data ID3, and clinical data ID4 of acute cerebral infarction based on a machine learning model AIM and output a result value of the probability prediction value OD for the recommended occurrence time of acute cerebral infarction. At this time, the processor 222 may generate machine learning-based reference data based on the first image and the second image. That is, the processor 222 may generate the reference data based on the machine learning model AIM based on the first image data ID1 corresponding to the fluid-attenuated inversion recovery image and the second image data ID2 corresponding to the diffusion-weighted image.

The machine learning model AIM may be constructed to learn various first image data ID1, second image data ID2, infarction region data ID3, and clinical data ID4 of acute cerebral infarction included in the input data through correlation. The machine learning model AIM may construct and perform reinforce learning as a learning data set various first image data ID1, various second image data ID2, various infarction region data ID3, and various clinical data ID4 of acute cerebral infarction using a learning algorithm. At this time, the memory 221 may store the probability prediction value OD for the occurrence time of acute cerebral infarction analyzed by learning based on the machine learning model AIM. Here, the clinical data ID4 of acute cerebral infarction may include at least one of various morphological findings, the patient's age at diagnosis, or the location of acute cerebral infarction.

An input module 230 may input the first image data corresponding to a fluid-attenuated inversion recovery image for the acute cerebral infarction patient, and the second image data corresponding to a diffusion-weighted image for the acute cerebral infarction patient. In addition, the input module 230 may input infarction region data for the acute cerebral infarction patient, and clinical data of acute cerebral infarction for the acute cerebral infarction patient. At this time, the clinical data of acute cerebral infarction may include at least one of various morphological findings, the patient's age at diagnosis, or the location of acute cerebral infarction. For example, the input module 230 may scan and input the first image data corresponding to a fluid-attenuated inversion recovery image, the second image data corresponding to a diffusion-weighted image, and the infarction region data, and any input means capable of inputting clinical data of acute cerebral infarction is possible.

The processor 222 may output the probability prediction value for the occurrence time of acute cerebral infarction based on the first image data, the second image data, and the infarction region data. In addition, the processor 222 may output the probability prediction value for the occurrence time of acute cerebral infarction based on the first image data, the second image data, the infarction region data, and the clinical data of acute cerebral infarction. In addition, the processor 222 may further label an area of the infarction region, perform machine learning-based learning based on the first image data, the second image data, and the area data of the infarction region, and further output the probability prediction value for the occurrence time of acute cerebral infarction analyzed by learning based on machine learning. In addition, the processor 222 may further label the area of the infarction region by location, perform machine learning-based learning based on the first image data, the second image data, and the area data of the infarction region by location, and further output the probability prediction value for the occurrence time of acute cerebral infarction analyzed by learning based on machine learning. In addition, the processor 222 may further label the area of the transition state by location of the infarction region, perform machine learning-based learning based on the first image data, the second image data, and the area data of the transition state by location of the infarction region, and further output the probability prediction value for the occurrence time of acute cerebral infarction that is learned and analyzed based on machine learning.

In this case, the processor 222 may standardize an intensity of the second image, perform machine learning-based learning based on the standardized second image, and detect the infarction region in the second image that is learned and analyzed based on machine learning. The second image corresponding to the standardized diffusion-weighted image may be output with improved image quality while increasing the readability when analyzed based on the machine learning model AIM by the processor 222.

The processor 222 may output the result value of the probability prediction value for the occurrence time of acute cerebral infarction that is recommended corresponding to the input information of the input module 230. A display module 240 may display the probability prediction value for the occurrence time of acute cerebral infarction output by the processor 222. For example, the display module 240 may display the probability prediction value belonging to each section of 0 to 3 hours, 3 to 4.5 hours, 4.5 to 6 hours, 6 to 12 hours, and more than 12 hours for the occurrence time of acute cerebral infarction.

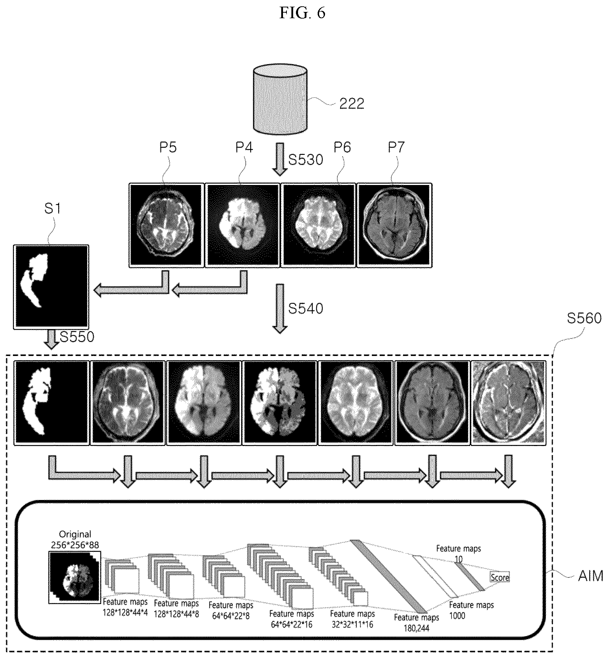

FIG. 5 is a flowchart illustrating a method for predicting an occurrence time of acute cerebral infarction according to the present disclosure. FIG. 6 is a diagram illustrating an example of a process for outputting a probability prediction value for an occurrence time of acute cerebral infarction in the output step of FIG. 5.

Referring to FIG. 5 and FIG. 6, a method for predicting an occurrence time of acute cerebral infarction may include a receiving step S510, a classification step S520, an acquisition step S530, a matching step S540, a detection step S550, and an output step S560.

In the receiving step, the images P1, P2, P3, ... of the acute cerebral infarction patient may be received from the imaging device 100 by the communication module 210 (step S510). At this time, the imaging device 100 may obtain the images P1, P2, P3, ... of the acute cerebral infarction patient. For example, the imaging device 100 may obtain magnetic resonance imaging (MRI) of the acute cerebral infarction patient.

In the classification step, images P1, P2, P3, ... of the acute cerebral infarction patient may be classified into the first image corresponding to a fluid-attenuated inversion recovery (FLAIR) image and the second image corresponding to a diffusion weighted image (DWI) by the processor 222 (step S520).

The acquisition step may acquire the first image corresponding to the classified fluid-attenuated inversion recovery image and the second image corresponding to the classified diffusion-weighted image by the processor 222 (step S530). At this time, the first image P4 corresponding to the fluid-attenuated inversion recovery image may gradually display the acute cerebral infarction range as time elapses after the occurrence of acute cerebral infarction. That is, the first image P4 corresponding to the fluid-attenuated inversion recovery image is the occurrence time of acute cerebral infarction, and the degree of expression of the infarction region of acute cerebral infarction varies depending on the elapsed time. In addition, the second image P5 corresponding to the diffusion-weighted image may display the acute cerebral infarction region within a short period of time after the occurrence of acute cerebral infarction, so that the acute cerebral infarction range may be immediately confirmed. Accordingly, the present disclosure may identify the occurrence time of acute cerebral infarction based on the discrepancy between the first image P4 corresponding to the fluid-attenuated inversion recovery image and the second image P5 corresponding to the diffusion-weighted image. At this time, the image of P6 may be the fluid-attenuated inversion recovery image of a normal person, and the image of P7 may be the diffusion-weighted image of a normal person.

Here, the processor 222 may generate the machine learning-based reference data based on the first image and the second image. That is, the processor 222 may generate the reference data based on the machine learning model AIM based on the first image data ID1 corresponding to the fluid-attenuated inversion recovery image and the second image data ID2 corresponding to the diffusion-weighted image. At this time, the first image and the second image may be acute cerebral infarction patients whose occurrence time of acute cerebral infarction is not confirmed.

The alignment step may align the first image and the second image to the MNI (Montreal Neulological Institute) region corresponding to the activated region of the brain by the processor 222 (step S540). The detection step may detect the infarct region in the second image by the processor 222 (step S550). At this time, the processor 222 may standardize the intensity of the second image, perform machine learning-based learning based on the standardized second image, and detect the infarct region in the second image analyzed by learning based on the machine learning. The second image corresponding to the standardized diffusion-weighted image may be output with improved image quality while increasing the readability when analyzed by learning based on the machine learning model AIM by the processor 222. Here, the MNI region may include the MNI coordinate value output as a result value for the activated region of the brain when analyzing the brain image. At this time, the infarction region may be a region where normal blood supply to the growing tissue is disrupted due to narrowing or blockage of blood vessels by thrombosis or embolism.

The output step may perform learning based on the machine learning model AIM by the processor 222 based on the first image data for the first image, the second image data for the second image, and the infarction region data for the infarction region, and output the probability prediction value for the occurrence time of acute cerebral infarction analyzed by learning based on the machine learning model AIM (step S560).

In addition, the output step may perform learning based on the machine learning model AIM by the processor 222 based on the first image data, the second image data, the infarction area data, and the clinical data of acute cerebral infarction, and output the probability prediction value for the occurrence time of acute cerebral infarction analyzed by learning based on the machine learning model AIM (step S560).

In addition, the output step may further label the area of the infarcted region by the processor 222, perform learning based on the machine learning model AIM based on the first image data, the second image data, and the area data of the infarcted region, and further output a probability prediction value for the occurrence time of acute cerebral infarction analyzed by learning based on the machine learning model AIM (step S560).

In addition, the output step may further label the area of the infarcted region by location by the processor 222, perform learning based on the machine learning model AIM based on the first image data, the second image data, and the area data of the infarcted region by location, and further output the probability prediction value for the occurrence time of acute cerebral infarction analyzed by learning based on the machine learning model AIM (step S560).

In addition, the output step may further label the area of the transition state by location of the infarction region by the processor 222, perform learning based on the machine learning model AIM based on the first image data, the second image data, and the area data of the transition state by location of the infarct region, and may further output the probability prediction value for the occurrence time of acute cerebral infarction analyzed by learning based on the machine learning model AIM (step S560).

In this case, the processor 222 may combine the images of the extracted infarction region obtained through preprocessing into four-dimensional data and use them as input data of a convolutional neural network (CNN) to proceed with learning. In this disclosure, since the convolutional neural network is used, information such as location information and volume of the infarct region that are lost when using the existing support vector machine or DNN may also be learned.

The output step may output the result value of the probability prediction value for the recommended occurrence time of acute cerebral infarction corresponding to the input information of the input module 230 by the processor 222 (step S560). The display module 240 may display the probability prediction value for the occurrence time of acute cerebral infarction output by the processor 222. For example, the display module 240 may display the probability prediction value that the occurrence time of acute cerebral infarction belongs to each section of 0 to 3 hours, 3 to 4.5 hours, 4.5 to 6 hours, 6 to 12 hours, and more than 12 hours.

Meanwhile, a computer program stored in a computer-readable storage medium may perform the following operations for performing the method for predicting the occurrence time of acute cerebral infarction performed by the prediction device 200 when executed on one or more processors.

The above operations may include: the operation of receiving the image of the acute cerebral infarction patient from the imaging device 100; the operation of classifying the image into the first image corresponding to a fluid-attenuated inversion recovery image and the second image corresponding to a diffusion-weighted image; and the operation of acquiring the first image and the second image.

Thereafter, the operations may include: the operation of aligning the first image and the second image with respect to an MNI region corresponding to an activated region of the brain; the operation of detecting the infarct region in the second image; and the operation of outputting the probability prediction value for the occurrence time of acute cerebral infarction based on the first image, the second image, and the infarct region;

Here, the output operation may perform machine learning-based learning based on the first image, the second image, and the infarct region, and output the probability prediction value for the occurrence time of acute cerebral infarction analyzed by learning based on machine learning.

In this case, the detection operation may standardize the intensity of the second image, perform machine learning-based learning based on the standardized second image, and detect the infarction region in the second image analyzed by learning based on machine learning. Here, the output operation may label the area of the infarction region, perform machine learning-based learning based on the first image, the second image, and the area of the infarction region, and output the probability prediction value for the occurrence time of acute cerebral infarction analyzed by learning based on machine learning.

In addition, the above operations may further include the operation of generating the machine learning-based reference data based on the first image and the second image;
Meanwhile, the present disclosure may determine whether the golden hour (4.5 hours) has elapsed when a thrombolytic agent may be administered, which is one of the treatment methods when cerebral infarction occurs. At this time, the elapsed time of the patient in the existing image for learning may be classified into the first group in the case of less than 4.5 hours, and the second group in the case of 4.5 hours or more. At this time, the present disclosure may utilize not only the image for learning, but also the elapsed time of the classified patient as learning data.

The present disclosure may classify a plurality of patients into a training group, a validation group, and a test group when generating the machine learning-based reference data based on the first image and the second image of the plurality of patients, and may perform machine learning-based learning through the first image and the second image of the patients included in the training group. In addition, the present disclosure may determine the machine learning-based learning form through the validation group, and may determine whether the test group is set as reference data by applying it to the machine learning model.

The present disclosure may extract first feature information in the first image. Here, the first feature information may be one or more feature elements extracted from a specific region of the image data. At this time, the present disclosure may extract the first feature information by applying at least one of a Gray Level Cooccurrence Matrix (GLCM), a Run-Length Matrix (i.e., a Gray Level Run-Length Matrix (GLRLM), and a Local Binary Pattern (LBP)) to the signal intensity and gradient of the infarction region.

The present disclosure may extract second feature information that may be acquired from the infarct region in the second image. Here, the second feature information may include information on the size or volume of the infarction region, and may include a mean, a standard deviation, a skewness, and a kurtosis calculated for each signal intensity, signal gradient, and LBP (Local Binary Pattern) map in the infarction region.

In this way, the present disclosure may provide information that allows medical staff to safely and quickly determine the occurrence time of acute cerebral infarction.

At least one component may be added or deleted in response to the performance of the components illustrated in FIGS. 1 and 2. In addition, it will be readily understood by those skilled in the art that the relative positions of the components may be changed in response to the performance or structure of the system.

Although FIG. 5 describes that multiple steps are executed sequentially, this is merely an example of explaining the technical idea of the present embodiment, and those with common knowledge in the technical field to which the present embodiment belongs may modify and change the order described in FIG. 5 without departing from the essential feature of the present embodiment, or may apply various modifications and variations by executing one or more of the multiple steps in parallel, so FIG. 5 is not limited to a sequential order.

Meanwhile, the disclosed embodiments may be implemented in the form of a recording medium that stores instructions executable by a computer. The instructions may be stored in the form of program codes, and when executed by a processor, may generate a program module to perform the operations of the disclosed embodiments. The recording medium may be implemented as a computer-readable recording medium.

A computer-readable recording medium includes all types of recording media that store instructions that may be decoded by a computer. For example, there may be a ROM (Read Only Memory), a RAM (Random Access Memory), a magnetic tape, a magnetic disk, a flash memory, an optical data storage device, and the like.

The disclosed embodiments have been described with reference to the attached drawings as described above. Those skilled in the art to which the present disclosure pertains will understand that the present disclosure may be implemented in different forms from the disclosed embodiments without changing the technical idea or essential features of the present disclosure. The disclosed embodiments are exemplary and should not be construed as limiting.

## Claims

1. A device for predicting an occurrence time of acute cerebral infarction, comprising:
a communication module configured to perform communication with an imaging device that captures an image of an acute cerebral infarction patient; and
a processor configured to control an operation related to prediction of the occurrence time of acute cerebral infarction,
wherein the processor is configured to:
receive the image of the acute cerebral infarction patient from the imaging device through the communication module,
classify the image into a first image corresponding to a fluid-attenuated inversion recovery image and a second image corresponding to a diffusion-weighted image,
acquire the first image and the second image,
align the first image and the second image with respect to a Montreal Neurological Institute, MNI, region corresponding to an activated region of a brain,
wherein the MNI region includes an MNI coordinate value output as a result value for the activated region of the brain when analyzing a brain image, detect an infarction region in the second image,
wherein the infarction region is an area where a blood vessel is narrowed or blocked by a blood clot or embolism,
label an area corresponding to a transition state for each position of an infarct region,
combine the first image, the second image, and data on the labeled area corresponding to the transition state for each position of the infarct region into four-dimensional data,
perform machine learning by using the four-dimensional data as input data of a convolutional neural network, CNN, and output, based on the machine learning, a probability prediction value for a time point of occurrence of acute cerebral infarction.

2. The device according to claim 1, wherein the processor is configured to:
standardize an intensity of the second image,
train a machine learning model based on the standardized second image, and
detect the infarction region in the second image using the trained machine learning model.

3. The device according to claim 1, wherein the processor is configured to:
further generate reference data based on the machine learning based on the first image and the second image,
wherein generating the reference data comprises:
classifying a plurality of patients into a training group, a validation group, and a test group.

4. A method for predicting an occurrence time of acute cerebral infarction performed by a prediction device, comprising:
receiving, by a communication module of the prediction device, an image of an acute cerebral infarction patient from an imaging device;
classifying, by a processor of the prediction device, the image into a first image corresponding to a fluid-attenuated inversion recovery image and a second image corresponding to a diffusion-weighted image;
acquiring, by the processor, the first image and the second image;
aligning, by the processor, the first image and the second image with respect to a Montreal Neurological Institute, MNI, region corresponding to an activated region of a brain,
wherein the MNI region includes an MNI coordinate value output as a result value for the activated region of the brain when analyzing a brain image;
detecting, by the processor, an infarction region in the second image,
wherein the infarction region is an area where a blood vessel is narrowed or blocked by a blood clot or embolism;
labeling, by the processor, an area corresponding to a transition state for each position of an infarct region;
combining, by the processor, the first image, the second image, and data on the labeled area corresponding to the transition state for each position of the infarct region into four-dimensional data;
performing, by the processor, machine learning by using the four-dimensional data as input data of a convolutional neural network, CNN, and outputting, by the processor, based on the machine learning, a probability prediction value for a time point of occurrence of acute cerebral infarction.

5. The method according to claim 4, wherein detecting the infarction region includes:
by the processor, standardizing an intensity of the second image,
training a machine learning model based on the standardized second image, and
detecting the infarction region in the second image using the trained machine learning model.

6. The method according to claim 4, further comprising:
by the processor, further generating reference data based on the machine learning based on the first image and the second image,
wherein generating the reference data comprises:
classifying a plurality of patients into a training group, a validation group, and a test group.

7. A system for predicting an occurrence time of acute cerebral infarction, comprising:
an imaging device configured to acquiring an image of an acute cerebral infarction patient; and
a prediction device for predicting the occurrence time of acute cerebral infarction configured to perform communication with the imaging device,
wherein the prediction device is configured to:
receive the image of the acute cerebral infarction patient from the imaging device,
classify the image into a first image corresponding to a fluid-attenuated inversion recovery image and a second image corresponding to a diffusion-weighted image,
acquire the first image and the second image,
align the first image and the second image with respect to a Montreal Neurological Institute, MNI, region corresponding to an activated region of a brain,
wherein the MNI region includes an MNI coordinate value output as a result value for the activated region of the brain when analyzing a brain image, detect an infarction region in the second image,
wherein the infarction region is an area where a blood vessel is narrowed or blocked by a blood clot or embolism,
label an area corresponding to a transition state for each position of an infarct region,
combine the first image, the second image, and data on the labeled area corresponding to the transition state for each position of the infarct region into four-dimensional data,
perform machine learning by using the four-dimensional data as input data of a convolutional neural network, CNN, and output, based on the machine learning, a probability prediction value for a time point of occurrence of acute cerebral infarction.

8. The system according to claim 7, wherein the prediction device is configured to:
standardize an intensity of the second image,
train a machine learning model based on the standardized second image, and
detect the infarction region in the second image using the trained machine learning model.

9. The system according to claim 7, wherein the prediction device is configured to:
further generate reference data based on the machine learning based on the first image and the second image,
wherein generating the reference data comprises:
classifying a plurality of patients into a training group, a validation group, and a test group.

## Patentansprüche

1. Vorrichtung zur Vorhersage eines Auftretenszeitpunkts eines akuten Hirninfarkts, die Folgendes umfasst:
ein Kommunikationsmodul, das dazu konfiguriert ist, mit einer Bildgebungsvorrichtung zu kommunizieren, die dazu konfiguriert ist, ein Bild eines Patienten mit akutem Hirninfarkt aufzunehmen; und
einen Prozessor, der dazu konfiguriert ist, einen Vorgang im Zusammenhang mit der Vorhersage des Auftretenszeitpunkts eines akuten Hirninfarkts zu steuern,
wobei der Prozessor für Folgendes konfiguriert ist:
Empfangen des Bilds des Patienten mit akutem Hirninfarkt von der Bildgebungsvorrichtung über das Kommunikationsmodul,
Klassifizieren des Bilds in ein erstes Bild, das einem Fluid-Attenuated-Inversion-Recovery-(FLAIR)-Bild entspricht, und ein zweites Bild, das einem
diffusionsgewichteten Bild entspricht,
Erfassen des ersten Bilds und des zweiten Bilds,
Ausrichten des ersten Bilds und des zweiten Bilds anhand eines Montreal Neurological Institute (MNI)-Bereichs, der einem aktivierten Bereich des Gehirns entspricht,
wobei der MNI-Bereich einen MNI-Koordinatenwert umfasst, der bei der Analyse eines Gehirnbilds als Ergebniswert für den aktivierten Bereich des Gehirns ausgegeben wird;
Erkennen eines Infarktbereichs im zweiten Bild,
wobei der Infarktbereich ein Bereich ist, in dem ein Blutgefäß durch ein Blutgerinnsel oder eine Embolie verengt oder blockiert ist,
Kennzeichnen eines Bereichs, der einem Übergangszustand entspricht, für jede Position eines Infarktbereichs,
Kombinieren des ersten Bilds, des zweiten Bilds und der Daten zum gekennzeichneten Bereich, der dem Übergangszustand für jede Position des Infarktbereichs entspricht, zu vierdimensionalen Daten,
Durchführen von maschinellem Lernen, indem die vierdimensionalen Daten als Eingabedaten für ein neuronales Faltungsnetzwerk (CNN) verwendet werden, und Ausgeben, auf der Grundlage des maschinellen Lernens, eines Wahrscheinlichkeitswerts für den Zeitpunkt des Auftretens eines akuten Hirninfarkts.

2. Vorrichtung nach Anspruch 1, wobei der Prozessor für Folgendes konfiguriert ist:
Standardisieren der Intensität des zweiten Bilds,
Trainieren eines Machine-Learning-Modells auf der Grundlage des standardisierten zweiten Bilds, und
Erkennen des Infarktbereichs im zweiten Bild mithilfe des trainierten Machine-Learning-Modells.

3. Vorrichtung nach Anspruch 1, wobei der Prozessor für Folgendes konfiguriert ist:
zusätzliches Erzeugen von Referenzdaten auf der Grundlage des maschinellen Lernens, basierend
auf dem ersten Bild und dem zweiten Bild,
wobei das Erzeugen der Referenzdaten Folgendes umfasst:
Klassifizieren einer Vielzahl von Patienten in eine Trainingsgruppe, eine Validierungsgruppe und eine Testgruppe.

4. Verfahren zur Vorhersage eines Auftretenszeitpunkts eines akuten Hirninfarkts, das von einer Vorhersagevorrichtung durchgeführt wird, das Folgendes umfasst:
Empfangen, durch ein Kommunikationsmodul der Vorhersagevorrichtung, eines Bilds eines Patienten mit akutem Hirninfarkt von einer Bildgebungsvorrichtung;
Klassifizieren, durch einen Prozessor der Vorhersagevorrichtung, des Bilds in ein erstes Bild, das einem Fluid-Attenuated-Inversion-Recovery-Bild entspricht, und ein zweites Bild, das einem diffusionsgewichteten Bild entspricht;
Erfassen, durch den Prozessor, des ersten Bilds und des zweiten Bilds;
Ausrichten, durch den Prozessor, des ersten Bilds und des zweiten Bilds in Bezug auf einen Montreal Neurological Institute (MNI)-
Bereich, der einem
aktivierten Bereich des Gehirns entspricht,
wobei der MNI-Bereich einen MNI-Koordinatenwert umfasst, der bei der Analyse eines Gehirnbilds als Ergebniswert für den aktivierten Bereich des Gehirns ausgegeben wird;
Detektieren, durch den Prozessor, eines Infarktbereichs in dem zweiten Bild,
wobei es sich bei dem Infarktbereich um einen Bereich handelt, in dem ein Blutgefäß durch ein Blutgerinnsel oder eine Embolie verengt oder blockiert ist;
Markieren, durch den Prozessor, eines Bereichs, der einem Übergangszustand entspricht, für jede Position eines Infarktbereichs;
Kombinieren, durch den Prozessor, des ersten Bilds, des zweiten Bilds und
von Daten über den markierten Bereich, der dem Übergangszustand für jede Position des Infarktbereichs entspricht, zu vierdimensionalen Daten;
Durchführen, durch den Prozessor, von maschinellem Lernen unter Verwendung der vierdimensionalen Daten als Eingabedaten für ein neuronales Faltungsnetzwerk (CNN) sowie
Ausgeben, durch den Prozessor, auf der Grundlage des maschinellen Lernens, eines Wahrscheinlichkeitswerts für den Zeitpunkt des Auftretens eines akuten Hirninfarkts.

5. Verfahren nach Anspruch 4, wobei das Detektieren des Infarktbereichs Folgendes beinhaltet:
durch den Prozessor, Standardisieren einer Intensität des zweiten Bilds, Trainieren eines Machine-Learning-Modells auf der Grundlage des standardisierten zweiten Bilds, und
Detektieren des Infarktbereichs in dem zweiten Bild unter Verwendung des trainierten Machine-Learning-Modells.

6. Verfahren nach Anspruch 4, das ferner Folgendes umfasst:
durch den Prozessor, weiteres Erzeugen von Referenzdaten auf Grundlage des maschinellen Lernens anhand des ersten Bilds und des zweiten Bilds,
wobei das Erzeugen der Referenzdaten Folgendes umfasst:
Klassifizieren einer Vielzahl von Patienten in eine Trainingsgruppe, eine Validierungsgruppe und eine Testgruppe.

7. System zur Vorhersage eines Auftretenszeitpunkts eines akuten Hirninfarkts, das Folgendes umfasst:
eine Bildgebungsvorrichtung, die zur Erfassung eines Bilds eines Patienten mit akutem Hirninfarkt ausgelegt ist; und
eine Vorhersagevorrichtung zur Vorhersage des Auftretenszeitpunkts eines akuten Hirninfarkts, die dazu konfiguriert ist, mit der Bildgebungsvorrichtung zu kommunizieren,
wobei die Vorhersagevorrichtung für Folgendes konfiguriert ist:
Empfangen des Bilds des Patienten mit akutem Hirninfarkt von der Bildgebungsvorrichtung, Klassifizieren des Bilds in ein erstes Bild, das einem Fluid-Attenuated-Inversion-Recovery-(FLAIR)-Bild entspricht, und ein zweites Bild, das einem
diffusionsgewichteten Bild entspricht,
Erfassen des ersten Bilds und des zweiten Bilds,
Ausrichten des ersten Bilds und des zweiten Bilds in Bezug auf einen Montreal Neurological Institute (MNI)-
Bereich, der einem aktivierten Bereich des Gehirns entspricht,
wobei der MNI-Bereich einen MNI-Koordinatenwert umfasst, der bei der Analyse eines Gehirnbilds als Ergebniswert für den aktivierten Bereich des Gehirns ausgegeben wird;
Detektieren eines Infarktbereichs im zweiten Bild,
wobei der Infarktbereich ein Bereich ist, in dem ein Blutgefäß durch ein Blutgerinnsel oder eine Embolie verengt oder blockiert ist,
Markieren eines Bereichs, der einem Übergangszustand entspricht, für jede Position eines Infarktbereichs,
Kombinieren des ersten Bilds, des zweiten Bilds und der Daten zum gekennzeichneten Bereich, der dem Übergangszustand für jede Position des Infarktbereichs entspricht, zu vierdimensionalen Daten,
Durchführen von maschinellem Lernen, indem die vierdimensionalen Daten als Eingabedaten für ein neuronales Faltungsnetzwerk (Convolutional Neural Network = CNN) verwendet werden, und Ausgeben, auf der Grundlage des maschinellen Lernens, eines Wahrscheinlichkeitswerts für den Zeitpunkt des Auftretens eines akuten Hirninfarkts.

8. System nach Anspruch 7, wobei die Vorhersagevorrichtung für Folgendes konfiguriert ist:
Standardisieren der Intensität des zweiten Bilds,
Trainieren eines Machine-Learning-Modells auf der Grundlage des standardisierten zweiten Bilds, und
Detektieren des Infarktbereichs im zweiten Bild mithilfe des trainierten Machine-Learning-Modells.

9. System nach Anspruch 7, wobei die Vorhersagevorrichtung für Folgendes konfiguriert ist:
weiteres Erzeugen von Referenzdaten auf der Grundlage des maschinellen Lernens anhand des ersten Bilds und des zweiten Bilds,
wobei das Erzeugen der Referenzdaten Folgendes umfasst:
Klassifizieren einer Vielzahl von Patienten in eine Trainingsgruppe, eine Validierungsgruppe und eine Testgruppe.

## Revendications

1. Dispositif destiné à prédire un temps de survenue d'un infarctus cérébral aigu, comprenant :
un module de communication configuré pour réaliser une communication avec un dispositif d'imagerie qui capture une image d'un patient souffrant d'un infarctus cérébral aigu ; et
un processeur configuré pour commander une opération associée à la prédiction du temps de survenue d'un infarctus cérébral aigu,
dans lequel le processeur est configuré pour :
recevoir l'image du patient souffrant d'un infarctus cérébral aigu en provenance du dispositif d'imagerie par l'intermédiaire du module de communication,
classifier l'image en une première image correspondant à une image d'atténuation fluidique par inversion-récupération et une seconde image correspondant à une image pondérée en diffusion,
acquérir la première image et la seconde image,
aligner la première et la seconde image par rapport à une région de l'institut neurologique de Montréal, INM, correspondant à une région activée d'un cerveau,
dans lequel la région INM inclut une valeur de coordonnées INM émise comme valeur de résultat pour la région activée du cerveau lors de l'analyse d'une image cérébrale, détecter une région d'infarctus dans la seconde image,
dans lequel la région d'infarctus est une zone où un vaisseau sanguin est rétréci ou obstrué par un caillot sanguin ou une embolie,
marquer une zone correspondant à un état de transition pour chaque position d'une région d'infarctus,
combiner la première image, la seconde image, et des données sur la zone marquée correspondant à l'état de transition pour chaque position de la région d'infarctus en données quadridimensionnelles,
réaliser un apprentissage automatique en utilisant les données quadridimensionnelles comme données d'entrée d'un réseau neuronal convolutif, CNN, et émettre, sur la base de l'apprentissage automatique, une valeur de prédiction de probabilité pour un point de survenue temporel d'un infarctus cérébral aigu.

2. Dispositif selon la revendication 1, dans lequel le processeur est configuré pour :
standardiser une intensité de la seconde image,
entraîner un modèle d'apprentissage automatique sur la base de la seconde image standardisée, et
détecter la région d'infarctus dans la seconde image en utilisant le modèle d'apprentissage automatique entraîné.

3. Dispositif selon la revendication 1, dans lequel le processeur est configuré pour :
générer en outre des données de référence sur la base de l'apprentissage automatique basé sur la première image et la seconde image,
dans lequel la génération des données de référence comprend :
la classification d'une pluralité de patients en un groupe d'entraînement, un groupe de validation et un groupe d'essai.

4. Procédé destiné à prédire un temps de survenue d'un infarctus cérébral aigu mis en œuvre par un dispositif de prédiction, comprenant :
la réception, par un module de communication du dispositif de prédiction, d'une image d'un patient souffrant d'un infarctus cérébral aigu en provenance d'un dispositif d'imagerie ;
la classification, par un processeur du dispositif de prédiction, de l'image en une première image correspondant à une image d'atténuation fluidique par inversion-récupération et en une seconde image correspondant à une image pondérée en diffusion ;
l'acquisition, par le processeur, de la première image et de la seconde image ;
l'alignement, par le processeur, de la première image et de la seconde image par rapport à une région de l'institut neurologique de Montréal, INM, correspondant à une région activée d'un cerveau,
dans lequel la région INM inclut une valeur de coordonnées INM émise comme valeur de résultat pour la région activée du cerveau lors de l'analyse d'une image cérébrale ;
la détection, par le processeur, d'une région d'infarctus dans la seconde image,
dans lequel la région d'infarctus est une zone où un vaisseau sanguin est rétréci ou obstrué par un caillot sanguin ou une embolie ;
le marquage, par le processeur, d'une zone correspondant à un état de transition pour chaque position d'une région d'infarctus ;
la combinaison, par le processeur, de la première image, de la seconde image, et de données sur la zone marquée correspondant à l'état de transition pour chaque position de la région d'infarctus en données quadridimensionnelles ;
la réalisation, par le processeur, d'un apprentissage automatique en utilisant les données quadridimensionnelles comme données d'entrée d'un réseau neuronal convolutif, CNN, et
l'émission, par le processeur, sur la base de l'apprentissage automatique, d'une valeur de prédiction de probabilité pour un point de survenue temporel d'un infarctus cérébral aigu.

5. Procédé selon la revendication 4, dans lequel la détection de la région d'infarctus inclut :
par le processeur, la standardisation d'une intensité de la seconde image, l'entraînement d'un modèle d'apprentissage automatique sur la base de la seconde image standardisée, et
la détection de la région d'infarctus dans la seconde image en utilisant le modèle d'apprentissage automatique entraîné.

6. Procédé selon la revendication 4, comprenant en outre :
par le processeur, la génération en outre de données de référence sur la base de l'apprentissage automatique basé sur la première image et la seconde image,
dans lequel la génération des données de référence comprend :
la classification d'une pluralité de patients en un groupe d'entraînement, un groupe de validation et un groupe d'essai.

7. Système destiné à prédire un temps de survenue d'un infarctus cérébral aigu, comprenant :
un dispositif d'imagerie configuré pour acquérir une image d'un patient souffrant d'un infarctus cérébral aigu ; et
un dispositif de prédiction pour prédire le temps de survenue d'un infarctus cérébral aigu configuré pour réaliser une communication avec le dispositif d'imagerie,
dans lequel le dispositif de prédiction est configuré pour :
recevoir l'image du patient souffrant d'un infarctus cérébral aigu en provenance du dispositif d'imagerie,
classifier l'image en une première image correspondant à une image d'atténuation fluidique par inversion-récupération et une seconde image correspondant à une image pondérée en diffusion,
acquérir la première image et la seconde image,
aligner la première image et la seconde image par rapport à une région de l'institut neurologique de Montréal, INM, correspondant à une région activée d'un cerveau,
dans lequel la région INM inclut une valeur de coordonnées INM émise comme valeur de résultat pour la région activée du cerveau lors de l'analyse d'une image cérébrale, détecter une région d'infarctus dans la seconde image,
dans lequel la région d'infarctus est une zone où un vaisseau sanguin est rétréci ou obstrué par un caillot sanguin ou une embolie,
marquer une zone correspondant à un état de transition pour chaque position d'une région d'infarctus,
combiner la première image, la seconde image, et des données sur la zone marquée correspondant à l'état de transition pour chaque position de la région d'infarctus en données quadridimensionnelles,
réaliser un apprentissage automatique en utilisant les données quadridimensionnelles comme données d'entrée d'un réseau neuronal convolutif, CNN, et émettre, sur la base de l'apprentissage automatique, une valeur de prédiction de probabilité pour un point de survenue temporel d'un infarctus cérébral aigu.

8. Système selon la revendication 7, dans lequel le dispositif de prédiction est configuré pour :
standardiser une intensité de la seconde image,
entraîner un modèle d'apprentissage automatique sur la base de la seconde image standardisée, et
détecter la région d'infarctus dans la seconde image en utilisant le modèle d'apprentissage automatique entraîné.

9. Système selon la revendication 7, dans lequel le dispositif de prédiction est configuré pour :
générer en outre des données de référence sur la base de l'apprentissage automatique basé sur la première image et la seconde image,
dans lequel la génération des données de référence comprend :
la classification d'une pluralité de patients en un groupe d'entraînement, un groupe de validation et un groupe d'essai.
